# EUROPEAN PATENT APPLICATION

(11) **EP 4 549 453 A1**
(43) Date of publication of application: **07.05.2025**
(21) Application number: 24206848.4
(22) Date of filing: 16.10.2024
(51) Int. Cl.: C07K 14/195, C12N 9/10, C12N 9/12, C12N 9/90, C12P 7/22, C12P 7/24, C12R 1/01

(54) **RECOMBINANT AMYCOLATOPSIS FOR IMPROVING VANILLIN YIELD, CONSTRUCTION METHOD AND APPLICATION THEREOF**

(30) Priority: 20.10.2023 CN 202311367050
(71) Applicant: Shaanxi Healthful Bioengineering Co., Ltd., Xian Shaanxi (CN)
(72) Inventor: QIANG, Shan, Xian (CN); YANG, Lu, Xian (CN); GUO, Jianqi, Xian (CN); MENG, Yonghong, Xian (CN); NIU, Yongjie, Xian (CN)
(74) Representative: Zaboliene, Reda

(57) **Abstract**

The present invention provides a method utilizing the deletion of the regulatory protein gene *tyrR1* and the overexpression of the *tyrA-aroF* genes to improve vanillin yield through fermentation with glucose as the substrate in *Amycolatopsis.* Additionally, the invention introduces a recombinant *Amycolatopsis* strain with a high vanillin yield, which has knocked out the regulatory protein gene *tyrR1* and the pyruvate kinase gene *pyk.* This strain also overexpresses the endogenous chorismate mutase/prephenate dehydrogenase bifunctional enzyme gene *tyrA,* the 3-deoxy-7-phosphoheptulonate synthase gene *aroF,* and the phosphoenolpyruvate synthase gene *ppsA.* This recombinant *Amycolatopsis* ferments glucose to produce vanillin, achieving a yield of 4.72 g/L, significantly higher than that of existing technologies.

## Description

### FIELD OF THE INVENTION

The present invention belongs to the technical field of genetic engineering. More specifically, it relates to a recombinant *Amycolatopsis* for improving the yield of vanillin, and it also involves the application of the *Amycolatopsis* in the production of vanillin.

### BACKGROUND OF THE INVENTION

Vanillin (3-methoxy-4-hydroxybenzaldehyde), also known as vanillic aldehyde, is one of the most widely used flavoring agents globally, with extensive applications in the food, pharmaceutical, cosmetic, and agricultural industries. The global demand for vanillin is currently around 20,000 tons. There are three main sources of vanillin in the market: (1) natural vanillin extracted from vanilla beans; (2) vanillin produced via chemical synthesis; and (3) vanillin produced through microbial transformation. Due to the limitations of plant extraction and chemical synthesis methods, people pay more and more attention to microbial transformation for vanillin production. In this method, eugenol, isoeugenol, and ferulic acid are the main substrates used for vanillin production. However, eugenol and isoeugenol are highly toxic, and ferulic acid is expensive. Therefore, making the search for safe and inexpensive raw materials to produce vanillin is an important research direction.

Glucose is an ideal raw material for biosynthesis of vanillin due to its low cost, abundant availability, and safety. Studies by Hansen et al. demonstrated that metabolically engineered yeast strains, including *Schizosaccharomyces pombe* and *Saccharomyces cerevisiae,* can produce 65 mg/L and 45 mg/L of vanillin using glucose as the substrate, respectively. However, yeast has a strong capacity for vanillin metabolism, leading to reduced yields and the formation of by-products. Chinese patent application CN 106032538A constructed a metabolically engineered *Escherichia coli* that produces vanillin through the phenylpropanoid pathway by introducing five exogenous genes, but the yield is low, and vanillin exhibited high toxicity to *E. coli,* hindering its accumulation. Patent application CN 114703113A achieved the biosynthesis of vanillin from glucose in *Amycolatopsis* by expressing the genes for tyrosine ammonia-lyase *(tal), p-*coumaric acid-3-hydroxylase *(sam5),* and caffeic acid O-methyltransferase *(com),* while deleting the gene for prephenate dehydratase, resulting in a vanillin yield of 1.25 g/L in shake flask fermentation. Although this patent enables the *de novo* synthesis of vanillin, the vanillin yield remains low, making it difficult to achieve industrial-scale production. To increase vanillin yield, the flux of tyrosine, an important precursor in vanillin synthesis, needs to be enhanced. Most research on tyrosine biosynthesis has been conducted in *E. coli,* and there are currently no reports on enhancing tyrosine production in *Amycolatopsis.*

### DETAILED DESCRIPTION OF THE INVENTION

The purpose of the present invention is to overcome the limitations of existing technologies by providing a genetically modified recombinant *Amycolatopsis* with high vanillin production.

To achieve this goal, the present invention provides an application of the deletion of regulatory protein gene *tyrR1* in *Amycolatopsis* sp. for improving the vanillin yield through fermentation with glucose as the substrate. The gene sequence of *tyrR1* is shown as SEQ ID NO:1 and its protein accession number is WP_020416143.1.

The present invention also provides an application of overexpressing *tyrA-aroF* genes and deleting regulatory protein *tyrR1* in *Amycolatopsis* for improving the vanillin yield through fermentation with glucose as the substrate. The *tyrA* encodes the chorismate mutase/prephenate dehydrogenase bifunctional enzyme with the nucleotide sequence shown as SEQ ID NO: 2 and the protein accession number WP_020419478.1. The *aroF* encodes 3-deoxy-7-phosphoheptulonate synthase with the nucleotide sequence shown as SEQ ID NO:3 and the protein accession number WP_020418838.1. The *tyrR1* is represented by SEQ ID NO:1 with protein accession number WP_020416143.1.

Furthermore, the present invention provides a recombinant *Amycolatopsis* with high vanillin yield. This recombinant strain has knocked out the regulatory protein gene *tyrR1* and the pyruvate kinase gene *pyk,* while overexpressing the endogenous chorismate mutase/prephenate dehydrogenase bifunctional enzyme gene *tyrA,* the 3-deoxy-7-phosphoheptulonate synthase gene *aroF,* and the phosphoenolpyruvate synthase gene *ppsA,* with the nucleotide sequences of genes *tyrR1, tyrA, aroF, pyk,* and *ppsA* shown as SEQ ID NO: 1, SEQ ID NO:2, SEQ ID NO:3, SEQ ID NO:4, and SEQ ID NO:5, respectively.

Additionally, the present invention also provides a method of constructing the recombinant *Amycolatopsis* described above. The method comprises the following steps:
(1) Construction of recombinant *Amycolatopsis* with *tyrR1* gene deletion
   PCR amplifying upstream and downstream homologous arms of *tyrR1* gene by using VA-F2/VA-R2 and VA-F3/VA-R3 as primers and *Amycolatopsis* genome as template, and simultaneously connecting the two obtained *tyrR1-*up-2500 and *tyrR1*-down-2500 fragments to the *Hind*III/*Eco*RI site of the plasmid pKG1132 by an Assembly kit to construct the plasmid pKG1132-*ZyrR1*-2500;
   transforming the obtained plasmid pKG1132-tyrRl-2500 into the starting strain of *Amycolatopsis* by using the intergeneric conjugation method, wherein the single colony that grows on the GYM solid medium with resistance to apramycin is the strain that has undergone homologous single exchange; carrying out subculture on the single-exchange strain in non-antibiotic GYM medium, wherein the strain that grows on non-antibiotic GYM plate but not on apramycin-resistant plate is the strain that has undergone homologous double exchange; conducting PCR verification on the double-exchange strain by using the genome of *Amycolatopsis* HM-145 as a control, wherein the correct strain is the recombinant *Amycolatopsis* with *tyrR1* gene deletion;
(2) Construction of recombinant *Amycolatopsis* integrating *tyrA* and *aroF* genes
   PCR amplifying the *permE* fragment by using VA-F1/VA-R1 as primers and the plasmid pKC1139-permE as template, and connecting the obtained *permE* fragment to the *Pvu*I/*Eco*RV site of the plasmid pSET152 by an Assembly kit to construct the plasmid pSET152-permE; PCR amplifying the *tyrA* gene using VA-F8/VA-R8 as primers and *Amycolatopsis* genome as template, connecting the obtained *tyrA* fragment to the *Bam*HI*lNsi*I site of the plasmid pSET152-permE by an Assembly kit to construct the plasmid pSET152-tyrA; PCR amplifying the *aroF* gene using VA-F10/VA-R10 as primers and *Amycolatopsis* genome as template, connecting the obtained *aroF* fragment to the *Bam*HI*lNsi*I site of the plasmid pSET152-permE by an Assembly kit to construct the plasmid pSET152-*aroF;*
   digesting pSET152-aro*F* with *Xba*I and *SpeI* and recovering the *permE-aroF* fragment from gel, then connecting the *permE-aroF* fragment to the *SpeI* site of pSET152-tyrA by T4 ligase to construct a plasmid *pSET152-tyrA-aroF;* digesting *pSET 152-tyrA-aroF* with *Xba*I and *SpeI* and recovering the *tyrA-aroF* fragment from gel, then connecting *tyrA-aroF* fragment to the *SpeI* site of pKG1132-tyrRl-2500 by T4 ligase to construct the plasmid pKG1132*-tyrR1-tyrA-aroF;*
   transforming the obtained plasmid *pKG1132-tyrRl-tyrA-aroF* into the recombinant *Amycolatopsis* with *tyrR1* gene deletion obtained in step (1) by using the intergeneric conjugation method, wherein the single colony that grows on the GYM solid medium with apramycin resistance is the strain that has undergone homologous single exchange; carrying out subculture on the single-exchange strain in the non-antibiotic GYM medium, wherein the strain that grows on the non-antibiotic GYM plate but not on apramycin-resistant plate is the strain that has undergone homologous double exchange; conducting PCR verifying on the double-exchange strain using the recombinant *Amycolatopsis* genome with *tyrR1* gene deletion in step (1) as a control, wherein the correct strain is the recombinant *Amycolatopsis* with *tyrR1* gene deletion and the *tyrA-aroF* gene integration;
(3) Construction of recombinant *Amycolatopsis with pyk* gene deletion
   PCR amplifying upstream and downstream homologous arms of *pyk* gene using VA-F11/VA-R11 and VA-F12/VA-R12 as primers and *Amycolatopsis* genome as template, and simultaneously connecting the two obtained pyk-up-2500 and pyk-down-2500 fragments to the *HindIII*/*EcoRI* site of the plasmid pKG1132 by an Assembly kit to construct the plasmid pKG1132-pyk-2500;
   transforming the plasmid pKG1132-pyk-2500 into the recombinant *Amycolatopsis* obtained in step (2) by using the intergeneric conjugation method, wherein the single colony that grows on the GYM solid medium with apramycin resistance is the strain that has undergone homologous single exchange; subculturing the single-exchange strain in the non-antibiotic GYM medium, wherein the strain that grows on the non-antibiotic GYM plate but not on the apramycin-resistant plate is the strain that has undergone homologous double exchange; conducting PCR verification on the double-exchange strain by using the recombinant *Amycolatopsis* genome obtained in step (2) as a control, wherein the correct strain is the recombinant *Amycolatopsis* with *tyrR1* and *pyk* gene deletion and the *tyrA-aroF* gene integration;
(4) Construction of recombinant *Amycolatopsis with ppsA* gene integrated at *pyk* site
   PCR amplifying the *ppsA* gene by using VA-F14/VA-R14 as primers and *Amycolatopsis* genome as template, connecting the *ppsA* fragment to the *Bam*HI*lNsi*I site of the plasmid pSET152-*permE* by an Assembly kit to construct the plasmid pSET152-ppsA; then digesting pSET152-ppsA with *Xbal* and *Spe*I*,* recovering the *ppsA* fragment from gel, and connecting the *ppsA* fragment to the *SpeI* site of pKG1132-pyk-2500 by T4 ligase to construct the plasmid pKG1132-*pyk ppsA;*
   transforming the obtained plasmid pKG1132-pyk-*ppsA* into the recombinant *Amycolatopsis* HM-151 by using the intergeneric conjugation method, wherein the single colony that grows on the GYM solid medium with apramycin resistance is the strain that has undergone homologous single exchange; subculturing the single-exchange strain in the non-antibiotic GYM medium, wherein the strain that grows on the non-antibiotic GYM plate but not on the apramycin-resistant plate is the strain that has undergone homologous double exchange; conducting PCR verification on the double-exchange strain by using the *Amycolatopsis* HM-151 genome as a control, wherein the correct strain is the recombinant *Amycolatopsis* with high vanillin yield. The strain is named HM-153.

In this invention, the starting strain used in step (1) is *Amycolatopsis* HM-145. This recombinant strain was constructed according to the description in Chinese Patent No. CN 114703113B. *Amycolatopsis* HM-145 expresses the tyrosine ammonia-lyase gene *(tal), p*-coumaric acid-3-hydroxylase (*sam5*)*,* and caffeic acid O-methyltransferase *(com),* while the prephenate dehydratase *(pheA)* is deleted. This enables the strain to synthesize vanillin through fermentation using glucose as the substrate, achieving a conversion of 25 g/L glucose to 1.25 g/L vanillin in a 250 mL Erlenmeyer flask containing 50 mL of M1 fermentation medium.

The present invention also provides the application of the above-mentioned recombinant *Amycolatopsis* or the recombinant *Amycolatopsis* obtained through the construction method described above for vanillin production.

In the present invention, the recombinant *Amycolatopsis* is inoculated into 50 mL of seed culture medium M1 and incubated at 30 °C and 200 rpm for 72 hours. The seed culture is then inoculated at 5% (v/v) into a 250 mL Erlenmeyer flask containing 50 mL of fermentation medium M1 and fermented at 37 °C and 200 rpm for 72 hours. The M1 medium contains 25 g/L glucose, 10 g/L yeast extract, 0.8 g/L sodium chloride, 5 g/L potassium dihydrogen phosphate, 0.2 g/L magnesium sulfate heptahydrate, 0.05 g/L calcium chloride, with the rest being water, and the pH adjusted to 7.2.

In this invention, by knocking out the regulatory protein gene *tyrR1* and the pyruvate kinase gene *pyk* in *Amycolatopsis,* and overexpressing the endogenous chorismate mutase/prephenate dehydrogenase bifunctional enzyme gene *tyrA, 3-*deoxy-7-phosphoheptulonate synthase gene *aroF,* and phosphoenolpyruvate synthase gene *ppsA,* the vanillin content reached 4.72 g/L. This represents a 277.6% increase in vanillin production compared to the starting strain HM-145. Given the low cost of the substrate and the high vanillin yield, this provides technical support for further improving vanillin fermentation yields in industrial production.

Through gene deletion screening, the present invention has confirmed that only the deletion of regulatory protein gene *tyrR1,* among *tyrR1, tyrR2,* and *tyrR3,* had the effect of increasing vanillin production in *Amycolatopsis.* Compared to the starting strain HM-145, the vanillin yield increased by 64.8%. Further, through gene expression screening, the sequence of the 3-deoxy-7-phosphoheptulonate synthase gene *aroF* is determined. Compared to *Amycolatopsis* HM-146, the vanillin yield of *Amycolatopsis* HM-150, which integrated the *tyrA-aroF* genes, increased by 69.42%.

### BRIEF DESCRIPTION OF THE DRAWINGS

Figure 1 is the strategy for the biosynthesis of vanillin in *Amycolatopsis.*
Figure 2 is the map of the plasmid pSET152-permE.

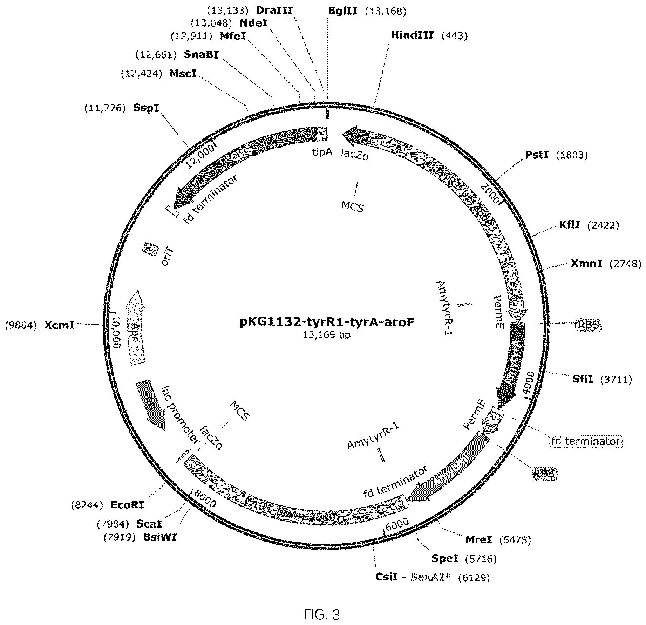

Figure 3 is the map of the plasmid *pKG1132-tyrRl-tyrA-aroF.*

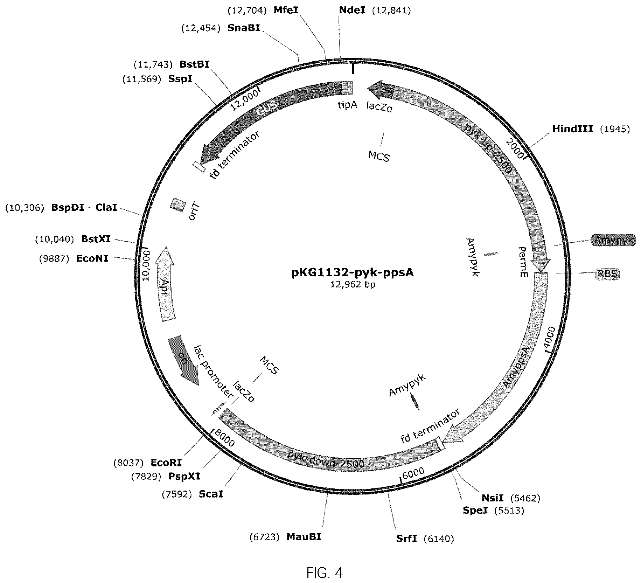

Figure 4 is the map of the plasmid pKG1132*-pyk-ppsA.*
Figure 5 is the PCR verification map of HM-150 strain. M, 1Kb DNAMarker; 1, 3 are the verification results of HM150 strain using TR1-F1/tyrA-R1 primers (3325bp); 2, 4 are the verification results of HM150 strain using *aroF*-F1/TR1-R2 primers (3174bp); 5 is the verification result of HM146 strain using TR1-F1/tyrA-R1 primers (no band); 6 is the verification result of HM146 strain using aroF-F1/TR1-R2 primers (no band).
Figure 6 is the PCR verification map of HM-154 strain. M, 1Kb DNA Marker; 1, 2, 3 are the verification results of HM154 strain using *pyk-*F1/*ppsA-*R1 primers (3253bp); 4 is the verification result of HM151 strain using *pyk-*F1/*ppsA-*R1 primers (no band); 5 is the verification result of HM151 strain using *ppsA-F1*/*pyk-*R2 primers (no band); 6, 7, 8 are the verification results of HM154 strain using *ppsA-F1*/*pyk-R2* primers (3302bp).

It will be able to understand the present invention better by the following examples. In the present invention, unless otherwise specified, "%" used to explain concentration refers to mass percentage, and ":" used to explain ratios refers to mass ratio.

The strains and plasmids used in the examples are listed in Table 1, and the sequences of the synthesized primers are listed in Table 2.

**Table 1 Strains and plasmids used in the study**

| Strains | Characteristic | Source |
|---|---|---|
| *Amycolatopsis* HM-145 | The starting strain, *Amycolatopsis* HM-141 strain with *pheA* gene knocked out and *tal, sam5* and *com* genes integrated | This laboratory (CN 114703113B) |
| *Amycolatopsis* HM-146 | HM-145, *tyrR1* gene knockout | This invention |
| *Amycolatopsis* HM-147 | HM-145, *tyrR2* gene knockout | This invention |
| *Amycolatopsis* HM-148 | HM-145, *tyrR3* gene knockout | This invention |
| *Amycolatopsis* HM-149 | HM-146, integration of *tyrA* and *aroG* genes at the *tyrR1* site | This invention |
| *Amycolatopsis* HM-150 | HM-146, integration of *tyrA* and *aroF* genes at the *tyrR1* site | This invention |
| *Amycolatopsis* HM-151 | HM-150, *pyk* gene knockout | This invention |
| *Amycolatopsis* HM-152 | HM-151, *tktA* gene integration | This invention |
| *Amycolatopsis* HM-153 | HM-151, *ppsA* gene integration | This invention |
| *Amycolatopsis* HM-154 | HM-151, integration of *ppsA* gene at the *pyk* site | This invention |
| DH5α | For plasmid cloning | Purchased from TransGen Biotech |
| ET12567 (pUZ8002) | Used as the donor for intergeneric conjugation between E. coli and *Amycolatopsis.* | Purchased from Shanghai Zeye |

| Plasmids | Characteristic | Source |
|---|---|---|
| pKC1139*-permE* | Plasmid pKC1139 with strong promoter *permE* | This laboratory (CN 114703113B) |
| pSET152 | Integrative expression vector, mainly used for gene screening | Purchased from MIAOLING BIOLOGY |
| pKG1132 | Integrative expression vectors, mainly used for gene knockout and gene integration | Barton N, et al. 2018 |
| *pSET 152-permE* | Plasmid pSET152 with strong promoter *permE* | This invention |
| pKG1132-*tyrR1*-2500 | The pKG1132 plasmid carries the upstream and downstream homology arms of the *tyrRl* gene deletion | This invention |
| pKG1132-*tyrR2*-2500 | The pKG1132 plasmid carries the upstream and downstream homology arms of the *tyrR2* gene deletion | This invention |
| pKG1132-*tyrR3*-2500 | The pKG1132 plasmid carries the upstream and downstream homology arms of the *tyrR3* gene deletion | This invention |
| pSET152-*tyrA* | pSET152-permE plasmid carries the *tyrA* gene | This invention |
| pSET152-aroG | pSET152-permE plasmid carries the *aroG* gene | This invention |
| pSET152-aroF | pSET152-permE plasmid carries the *aroF* gene | This invention |
| pSET152-*tyrA-aroG* | pSET152-permE plasmid carries the *tyrA* and *aroG* genes | This invention |
| pSET152*-tyrA-aroF* | pSET152-*permE* plasmid carries the *tyrA* and *aroF* genes | This invention |
| pKG1132*-tyrR1-tyrA-aroG* | pKG1132*-tyrR1*-2500 plasmid carries the *tyrA* and *aroG* genes | This invention |
| pKG 1132-*tyrR1-tyrA-aroF* | pKG1132-*tyrR1*-2500 plasmid carries the *tyrA* and *aroF* genes | This invention |
| pKG1132-*pyk*-2500 | The pKG1132 plasmid carries the upstream and downstream homology arms of the *pyk* gene deletion | This invention |
| pSET152-*tktA* | pSET152-permE carries the *tktA* gene | This invention |
| pSET152-*ppsA* | pSET152-*permE* carries the *ppsA* gene | This invention |
| pKG1132-*pyk-ppsA* | pKG1132-*pyk-*2500 carries the *ppsA* gene | This invention |

**Table 2 Primers used in the study**

| Primer name | Sequence 5'-3' | Application |
|---|---|---|
| VA-F1 | CTGTTGGGAAGGGCGTCTAGAAGCCCGACCCGAGCAC | |
| VA-R1 | GATTACGAATTCGATACTAGTACCGATACAATTAAAGGCTCCTTTT G | pSET152-*permE* |
| VA-F2 | GGCCAGTGCCAAGCTTGGGTTGCGGTCGACCACGATCA | |
| VA-R2 | CCTTGATCACTAGTGGACCGCCCGCTGATCCACCAT | pKG1132-*tyrRl-2500* |
| VA-F3 | CACTAGTGATCAAGGTGCCGCGCCGGTAG | |
| VA-R3 | ACATGATTACGAATTCTGGGATAGCACCGGAGGAGGGT | |
| VA-F4 | GGCCAGTGCCAAGCTTGTCCGGATCGGCAGTCCGGAGT | |
| VA-R4 | TGACGATCACTAGTCCCGGTCGTGGTCCACTCTGAC | pKG1132-*tyrR2-2500* |
| VA-FS | GACTAGTGATCGTCACGCCGGTGCACTGA | |
| VA-RS | ACATGATTACGAATTCCTGGAGGGGAACCTGTGCCGTT | |
| VA-F6 | GGCCAGTGCCAAGCTTGTACCCGCGGAGCATCCGCTGT | |
| VA-R6 | GCAGCACCACTAGTTCGAACCCGTCGACGCCCATGT | pKG1132-*tyrR3-*2500 |
| VA-F7 | AACTAGTGGTGCTGCGGATCCGCGTGTGA | |
| VA-R7 | ACATGATTACGAATTTGATGGCAAACGCGTCGGCGATGA | |
| VA-F8 | CCGGTTGGTAGGATCCAAGGAGGCAACAAGGTGATCGGGCTCGG GTTGATCG | pSET152-*tyrA* |
| VA-R8 | TTTTTGGAGATTTTATGCATTTACGGCACCTCGCCGATGGCG | |
| VA-F9 | CCGGTTGGTAGGATCCAAGGAGGCAACAAGGTGAACTGGACCGT GGACGTGC | pSET152*-aroG* |
| VA-R9 | TTTTTGGAGATTTTATGCATTCAACCCCGCAACATCTCCGCC | |
| VA-F10 | CCGGTTGGTAGGATCCAAGGAGGCAACAAGATGTCTCCCAGCGC CTCGACAC | pSET152*-aroF* |
| VA-R10 | TTTTTGGAGATTTTATGCATCTACCGCCGAGCAGCCACAGCC | |
| VA-F11 | CGGCCAGTGCCAAGCTGGGCAAGTACGAGCAGGGCATC | |
| VA-R11 | CTCACCGAACTAGTATCTTCGCGCGTCGGCTCACGT | pKG1132*-pyk-*2500bp |
| VA-F12 | TACTAGTTCGGTGAGGACGACCACGCCTGA | |
| VA-R12 | ACATGATTACGAATTCCACCGGGGCAAACGGCTCCACA | |
| VA-F13 | CCGGTTGGTAGGATCCAAGGAGGCAACAAGGTGTCCGATATCGC CTCCATCA | pSET152-*tktA* |
| VA-R13 | TTTTTGGAGATTTTATGCATTCAGGAGTTCTTGGTCTTGGCG | |
| TR1-F1 | TCAGGTCCAGCACCTCCTCCACCT | *tyrR1* gene deletion verification primers |
| TR1-R1 | GCAGCTCGGTCTCCCCGTTGATCGT | |
| TR1-F2 | TGCACCGGTGACGGTAGGTAGCCGGA | |
| TR1-R2 | AGGCGCTGATGGGGAGGGGGTGAT | |
| TR2-F1 | AGTACTTGAGCGTGGCCGGTTCGAC | *tyrR2* gene |
| TR2-R1 | TCCGGATTGGACGGTCACGGATCTC | deletion verification primers |
| TR2-F2 | AATTTGAGACGCGGTGCGACCGGCT | |
| TR2-R2 | TTCCGCGAGGAACACGGCCTGCAGT | |
| TR3-F1 | CAGTGGATCGACTGCACCACCGACGT | *tyrR3* gene deletion verification primers |
| TR3-R1 | AGCTGTCCACCCGTGGATGGAGAAC | |
| TR3-F2 | TGGAGAGCCACCTCGACCTGATCAC | |
| TR3-R2 | TACTGCGGGTTGGACGAGCCGAACA | |
| *pvA-*F1 | TACGACTACTTCGGCGGCTTCAGCC | *pyk* gene deletion verification primers |
| *pyk-*R1 | ATGAAGTACGCGTGCAGCGAGTGCAC | |
| *pyk-F2* | CACCATCGTGGGTGAACTAGCCGCT | |
| *pyk-R2* | AGCTCTCGGCGGACACGTGCAGCTCA | |
| *tyrA*-R1 | TGTCGTCCTCGACGCACACCACCCA | *tyrA-aroG*/*tyrA-aroF* gene integration verification |
| *aroF-*F1 | AGCCACGTGTTCGCGGGCATCAACA | |
| *aroG-*F1 | ATCGGCGTGAAGATCGGCCCGACC | primers |
| *ppsA-*R1 | AAGTCCCGCATGCGGTCGCAGTGCT | *ppsA* gene integration verification |
| *ppsA-*F1 | AGCACAACCCGATGATCGGCTACCG | primers |

The present invention involves the following culture medium:
the formula of the LB culture medium is as follows: 10g/L peptone, 5g/L yeast extract and 10g/L sodium chloride.
the formula of the GYM culture medium is as follows: 4g/L glucose, 4g/L yeast extract and 10g/L malt extract.
the formula of the GYM solid culture medium is as follows: 4g/L glucose, 4g/L yeast extract, 10g/L malt extract, 2g/L calcium carbonate and 20 g/L agar powder.
the formula of the M1 culture medium is as follows: 25g/L glucose, 10g/L yeast extract, 0.8g/L sodium chloride, 5g/L potassium dihydrogen phosphate, 0.2g/L magnesium sulfate heptahydrate, 0.05g/L calcium chloride, with the rest being water, and the pH adjusted to 7.2.

The conjugation transfer experiment used in the following examples includes the following steps:
*(1) Amycolatopsis* sp. (or other engineered strains) were activated on GYM solid medium and cultured at 30 °C for 3-4 days until colonies form. Then, the strain was inoculated into 50 mL of GYM liquid medium and cultured at 30 °C and 200 rpm for 2 days.
(2) The constructed plasmid was introduced into *E. coli* ET12567 (pUZ8002) via heat shock, and the cells were plated onto LB solid medium containing 25 µg/mL chloramphenicol, 25 µg/mL kanamycin, and 50 µg/mL apramycin. The plate was incubated at 37 °C for 12 hours until single colonies appear. A single colony was inoculated into 4 mL LB liquid medium containing the same antibiotics and cultured at 37 °C and 200 rpm overnight. Then, the culture was inoculated at 1% into 20 mL LB and grown at 37 °C and 200 rpm for 4-5 hours until the OD600 reached 0.4-0.6.
(3) 2 mL of *Amycolatopsis* culture and 1 mL of *E. coli* ET12567 (pUZ8002) carrying the target plasmid were collected respectively, centrifuged at 5000 g for 1 minute, and washed twice with non-antibiotic LB. Then, 100 µL of non-antibiotic LB medium is added. The *Amycolatopsis* and *E. coli* cultures were mixed at a volume ratio of 7:1. Then, 30 µL of the mixed cell suspension was spotted onto non-antibiotic GYM solid medium and incubated at 30 °C for 14 hours.
(4) The resulting colonies were scraped and spread on GYM solid medium containing 50 µg/mL apramycin and 25 µg/mL nalidixic acid. The plate is incubated at 30 °C for 4 days until single colonies appear, which are then verified by PCR.

### Example 1: Construction of Amycolatopsis with tyrR gene deletion

The sequences of the regulatory proteins *tyrR1, tyrR2* and *tyrR3* in *Amycolatopsis* sp. were aligned using the gene sequence of *Amycolatopsis* sp. ATCC 39116 from the NCBI website. The gene sequence of *tyrR1* is shown as SEQ ID NO: 1, with the protein accession number WP_020416143.1. The gene sequence of *tyrR2* is shown as SEQ ID NO: 6, with the protein accession number WP_020422712.1. The gene sequence of *tyrR3* is shown as SEQ ID NO: 7, with the protein accession number WP_020419034.1.

### 1. Construction of plasmid pKG1132-tyrR1-2500, pKG1132-tyrR2-2500, pKG1132-tyrR3-2500 with tyrR gene knockout

The upstream and downstream homologous arms of *tyrR1* gene of about 2.5 kb each were amplified by PCR using VA-F2/VA-R2 and VA-F3/VA-R3 as primers and *Amycolatopsis* genome as template. The obtained two fragments *tyrRl-up-2500* and *tyrR1-down-2500* were ligated into the *HindIII*/*EcoRI* site of the pKG1132 plasmid (Nadja Barton, et al., Enabling the valorization of guaiacol-based lignin: Integrated chemical and biochemical production of cis,cis-muconic acid using metabolically engineered Amycolatopsis sp ATCC 39116, Metabolic Engineerings 45 (2018) 200-210) by an Assembly kit to construct the plasmid pKG 1132-tyrRl-2500.

The upstream and downstream homologous arms of *tyrR2* gene of about 2.5 kb each were amplified by PCR using VA-F4/VA-R4 and VA-FS/VA-RS as primers and *Amycolatopsis* genome as template. The obtained two fragments *tyrR2*-up-2500 and *tyrR2*-down-2500 were ligated into the *HindIII*/*EcoRI* site of the pKG1132 plasmid by an Assembly kit to construct the plasmid pKG1132-*tyrR2-2500.*

The upstream and downstream homologous arms of *tyrR3* gene of about 2.5 kb each were amplified by PCR using VA-F6/VA-R6 and VA-F7/VA-R7 as primers and *Amycolatopsis* genome as template. The obtained two fragments *tyrR3*-up-2500 and tyrR3-down-2500 were ligated into the *HindIII*/*EcoRI* site of the pKG1132 plasmid by an Assembly kit to construct the plasmid pKG1132-*tyrR3-2500.*

### 2. Construction of Amycolatopsis with tyrR gene deletion

The plasmids pKG1132-tyrRl-2500, pKG1132-*tyrR2*-2500, and pKG1132-*tyrR3-2500* were transformed into *Amycolatopsis* HM-145 by using the intergeneric conjugation method, respectively. The single colony that grew on the GYM solid medium with apramycin resistance (final concentration of 50 µg/mL) was the strain that had undergone homologous single exchange. The single-exchange strain was subcultured in the non-antibiotic GYM medium. The strain that grew on the non-antibiotic GYM plate but not on the apramycin resistance plate was the strain that had undergone homologous double exchange. The double-exchange strain was verified by PCR using the genome of *Amycolatopsis* HM-145 as a control. The primers for verifying the deletion of *tyrR1* gene were TR1-F1/TR1-R1 and TR1-F2/TR1-R2. The primers for verifying the deletion of *tyrR2* gene were TR2-F1/TR2-R1 and TR2-F2/TR2-R2. The primers for verifying the deletion of *tyrR3* gene were TR3-F1/TR3-R1 and TR3-F2/TR3-R2. The correct bands verified by PCR were sent for sequencing, and the correct sequencing strain was the *Amycolatopsis* with tyrR gene deletion. The strains with the *tyrR1, tyrR2,* and *tyrR3* gene knockout were named HM-146, HM-147, and HM-148, respectively.

### 3. Fermentation comparison of the starting strain Amycolatopsis HM-145, HM-146, HM-147, and HM-148

Fermentation experiments were conducted using the *Amycolatopsis* HM-145, HM-146, HM-147, and HM-148 strains, respectively. The experimental methods are as follows:
The strains were inoculated into 50 mL seed culture medium M1 and incubated at 30 °C and 200 rpm for 72 hours. The seed culture was then used to inoculate 250 mL Erlenmeyer flask containing 50 mL fermentation medium M1 at a 5% inoculum ratio and fermented at 37°C and 200 rpm for 72 hours. After fermentation, the concentration of vanillin in the fermentation broth was measured.

The method for determining the production of vanillin is based on the method documented in Chinese invention patent application CN 113789292A, with results shown in Table 3.

**Table 3 Shake flask fermentation results of Amycolatopsis HM-145 and its engineered strains**

| Strains | Vanillin concentration (g/L) |
|---|---|
| *Amycolatopsis* HM-145 | 1.25 |
| HM-146 | 2.06 |
| HM-147 | 1.31 |
| HM-148 | 1.08 |

The results above indicated that the *tyrR1* gene-deleted strain HM-146 increased the yield of vanillin synthesized from glucose, rising from 1.25 g/L in the starting strain HM-145 to 2.06 g/L, an increase of 64.8%. The vanillin yield of the *tyrR2* gene-deleted strain HM-147 showed only a 4.8% increase compared to strain HM-145. In contrast, the vanillin yield of the *tyrR3* gene-deleted strain HM-148 decreased by 13.6% compared to strain HM-145. Thus, although *tyrR1, tyrR2,* and *tyrR3* are all regulatory protein genes in *Amycolatopsis,* only the knockout of the *tyrR1* gene significantly enhances vanillin production from glucose, while *tyrR2* and *tyrR3* do not exhibit similar effects.

Therefore, further operations were performed based on the *tyrR1* gene knockout strain *Amycolatopsis* HM-146.

### Example 2: Construction of Amycolatopsis HM-150 with tyrA and aroF genes integration

The sequences of the chorismate mutase/prephenate dehydrogenase bifunctional enzyme gene *tyrA,* 3-deoxy-7-phosphoheptulonate synthase enzyme gene *aroG* and *aroF* in *Amycolatopsis* sp. were aligned using the gene sequence of *Amycolatopsis* sp. ATCC 39116 from the NCBI website. The gene sequence of *tyrA* is shown as SEQ ID NO: 2, with the protein accession number WP_020419478.1. The gene sequence of *aroF* is shown as SEQ ID NO: 3, with the protein accession number WP_020418838.1. The gene sequence of *aroG* is shown as SEQ ID NO: 8, with the protein accession number WP_026153352.1.

### 1. Construction of integration plasmids pSET152-tyrA-aroG, pSET152-tyrA-aroF, pKG1132-tyrR1-tyrA-aroG and pKG1132-tyrR1-tyrA-aroF

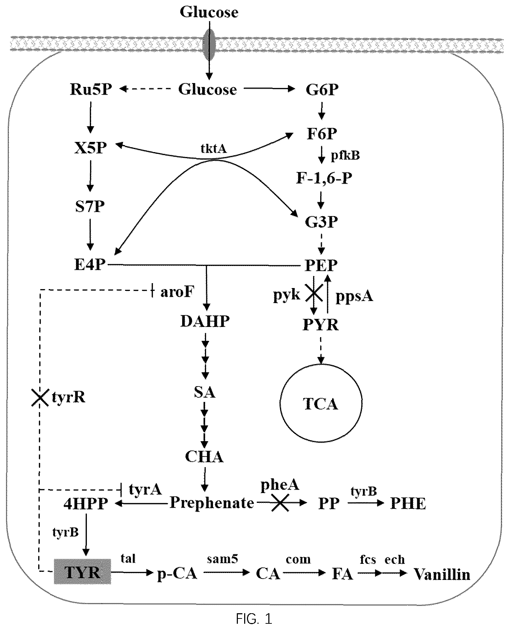

The *permE* fragment was amplified by PCR using VA-F1/VA-R1 as primers and the plasmid pKC1139-*permE* (constructed according to the description of Chinese patent No. CN 114703113B) as template. The obtained *permE* fragment was ligated into the *Pvu*I/*Eco*RV site of the plasmid pSET152 by an Assembly kit to construct the plasmid pSET152-*permE*. The plasmid map of pSET152*-permE* is shown in Figure 1.

The *tyrA* gene was amplified by PCR using VA-F8/VA-R8 as primers and *Amycolatopsis* genome as template. The *tyrA* fragment was ligated into the *Bam*HI/*Nsi*I site of the plasmid pSET152-*permE* by an Assembly kit to construct the plasmid pSET152-*tyrA*.

The *aroG* gene was amplified by PCR using VA-F9/VA-R9 as primers and *Amycolatopsis* genome as template. The *aroG* fragment was ligated into the *Bam*HI*lNsi*I site of the plasmid pSET152-*permE* by an Assembly kit to construct the plasmid pSET152-aroG.

The *aroF* gene was amplified by PCR using VA-F10/VA-R10 as primers and *Amycolatopsis* genome as template. The *aroF* fragment was ligated into the *Bam*HI/*Nsi*I site of the plasmid pSET152-*permE* by an Assembly kit to construct the plasmid pSET152-aroF.

pSET152-aroG was digested with *Xba*I and *SpeI* and recovered the *permE-aroG* fragment from gel. The *permE-aroG* fragment was ligated to the *SpeI* site of pSET152-tyrA by T4 ligase to construct the plasmid *pSET152-tyrA-aroG.*

pSET152-aroF was digested with *Xba*I and *SpeI* and recovered the *permE-aroF* fragment from gel. The *permE-aroF* fragment was ligated to the *SpeI* site of pSET152-tyrA by T4 ligase to construct the plasmid *pSET152-tyrA-aroF.*

pSET152*-tyrA-aroG* was digested with *Xba*I and *SpeI* and recovered the *tyrA-aroG* fragment from gel. The *tyrA-aroG* fragment was ligated to the *SpeI* site of pKG1132-tyrRl-2500 by T4 ligase to construct the plasmid pKG1132-*tyrR1-tyrA-aroG.*

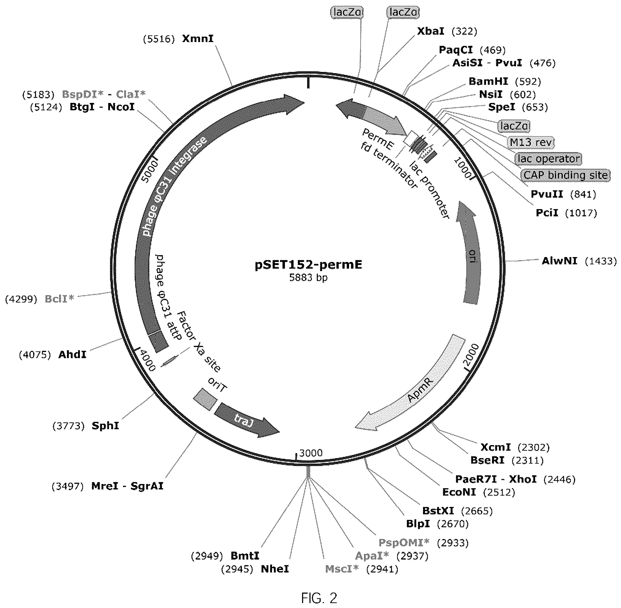

pSET152*-tyrA-aroF* was digested with *Xba*I and *SpeI* and recovered the *tyrA-aroF* fragment from gel. The *tyrA-aroF* fragment was ligated to the *SpeI* site of pKG1132-*tyrR1*-2500 by T4 ligase to construct the plasmid pKG1132-*tyrR1-tyrA-aroF.* The plasmid map of pKG1132*-tyrR1-tyrA-aroF* is shown in Figure 2.

### 2. Construction of Amycolatopsis HM-149 and HM-150 integrating tyrA-aroGltyrA-aroF genes

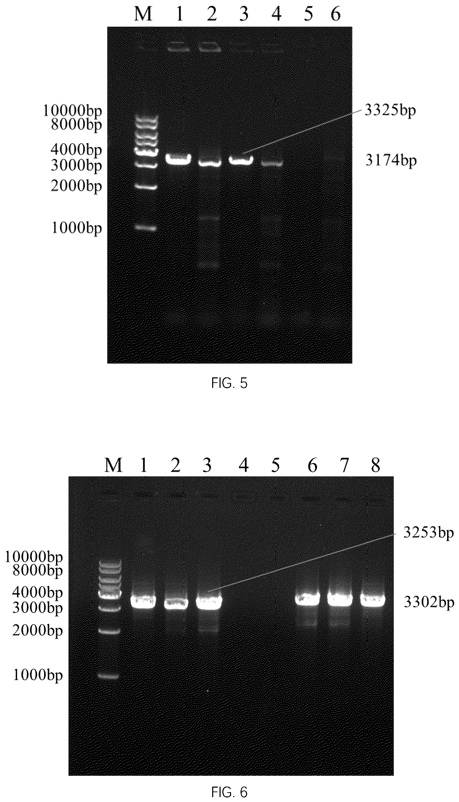

The plasmids pKG1132*-tyrR1-tyrA-aroG* and *pKG1132-tyrRl-tyrA-aroF* were transformed into *Amycolatopsis* HM-146 by using the intergeneric conjugation method, respectively. The single colony that grew on the GYM solid medium with apramycin resistance (final concentration of 50 µg/mL) was the strain that had undergone homologous single exchange. The single-exchange strain was subcultured in the non-antibiotic GYM medium. The strain that grew on the non-antibiotic GYM plate but not on the apramycin resistance plate was the strain that had undergone homologous double exchange. The double-exchange strain was verified by PCR using the genome of *Amycolatopsis* HM-146 as a control. The primers for verifying the deletion of *tyrR1* gene were TR1-F1/TR1-R1 and TR1-F2/TR1-R2. The primers for verifying the integration of *tyrA-aroG* gene were TR1-F1/*tyrA*-R1 and *aroG*-F1/TR1-R2. The primers for verifying the integration of *tyrA-aroG* gene were TR1-F1/*tyrA*-R1 and *aroG*-F1/TR1-R2. The primers for verifying the integration of *tyrA-aroF* gene were TR1-F1/*tyrA*-R1 and aroF-F1/TR1-R2. The correct bands verified by PCR were sent for sequencing, and the correct sequencing strain was the *Amycolatopsis* HM-149 and HM-150 with the integrated *tyrA-aroG*/*tyrA-aroF* gene. The PCR verification results of the HM-150 strain are shown in Figure 5.

### 3. Fermentation comparison of the strains HM-146, HM-149, and HM-150

Fermentation experiments were conducted using *Amycolatopsis* HM-146, HM-149 and HM-150 strains, respectively, following the same fermentation experimental methods as in Example 1. The fermentation results are shown in Table 4.

**Table 4 Shake flask fermentation results of Amycolatopsis HM-146, HM-149 and HM-150**

| Strains | Vanillin concentration (g/L) |
|---|---|
| *Amycolatopsis* HM-146 | 2.06 |
| HM-149 | 2.53 |
| HM-150 | 3.49 |

The results above indicated that, compared to *Amycolatopsis* HM-146, *Amycolatopsis* HM-149 with the integrated *tyrA-aroG* genes showed a 22.81% increase in vanillin production, while the strain HM-150 with the integrated *tyrA-aroF* gene exhibited a 69.42% increase in vanillin production. This results demonstrates that although both *aroG* and *aroF* are genes for 3-deoxy-7-phosphoheptulonate synthase, the expression of the *aroF* gene has a significant effect on enhancing vanillin production in *Amycolatopsis* when glucose is used as the substrate. In contrast, while *aroG* can also enhance vanillin production to some extent, the increase is not as substantial as expected.

Therefore, subsequent operations were carried out based on the strain HM-150 with the integrated *tyrA-aroF* genes.

### Example 3: Construction of Amycolatopsis with pyk gene deletion

To further increase vanillin production from glucose fermentation in *Amycolatopsis,* the sequence of the pyruvate kinase gene *pyk* was aligned using the gene sequence of *Amycolatopsis* sp. ATCC 39116 from the NCBI website. The gene sequence of *pyk* is shown as SEQ ID NO: 4, with the protein accession number WP_027935993.1.

### 1. Construction of plasmid pKG1132-pyk-2500 with pyk gene knockout

The upstream and downstream homologous arms of *pyk* gene of about 2.5 kb each were amplified by PCR using VA-F11/VA-R11 and VA-F12/VA-R12 as primers and *Amycolatopsis* genome as template. The obtained two pyk-up-2500 and pyk-down-2500 fragments were ligated into the *HindIII*/*EcoRI* site of the pKG1132 plasmid by an Assembly kit to construct the plasmid pKG1132-pyk-2500.

### 2. Construction of Amycolatopsis HM-151 with tyrR gene deletion

The plasmid pKG1132-pyk-2500 was transformed into *Amycolatopsis* HM-150 using the intergeneric conjugation method. The single colony that grew on the GYM solid medium with apramycin resistance was the strain that had undergone homologous single exchange. The single-exchange strain was subcultured in the non-antibiotic GYM medium. The strain that grew on the non-antibiotic GYM plate but not on the apramycin resistance plate was the strain that had undergone homologous double exchange. The double-exchange strain was verified by PCR using the genome of *Amycolatopsis* HM-150 as a control. The primers for verifying the deletion of *pyk* gene were *pyk-*F1/*pyk-*R1 and *pyk-*F2/pyk-R2. The correct bands verified by PCR were sent for sequencing, and the correct sequencing strain was the *Amycolatopsis* HM-151 with *pyk* gene deletion.

### 3. Fermentation comparison of the strains HM-150 and HM-151

Fermentation experiments were conducted using *Amycolatopsis* HM-150 and HM-151 strains, respectively, following the same fermentation experimental methods as in Example 1. The fermentation results are shown in Table 5.

**Table 5 Shake flask fermentation results of Amycolatopsis HM-150 and HM-151 strains**

| Strains | Vanillin concentration (g/L) |
|---|---|
| *Amycolatopsis* HM-150 | 3.49 |
| HM-151 | 3.85 |

The results indicated that the *pyk* gene deletion strain HM-151 increased the yield of vanillin by 10.32% compared to strain HM-150. Therefore, subsequent operations were performed based on the *pyk* gene-deleted strain HM-151.

### Example 4: Construction of Amycolatopsis HM-151 with ppsA gene integration

To further enhance the yield of vanillin produced by *Amycolatopsis* using glucose as a substrate, the sequences of the transketolase gene *tktA* and the phosphoenolpyruvate synthase gene *ppsA* in *Amycolatopsis* were compared based on the gene sequences of *Amycolatopsis* sp. ATCC 39116 from the NCBI website. The gene sequence of *ppsA* is shown as SEQ ID NO:5, with the protein accession number WP_020420172.1.

### 1. Construction of integration plasmids pSET152-tktA, pSET152-ppsA, pKG1132-pyk-ppsA

The *tktA* gene was amplified by PCR using VA-F13/VA-R13 as primers and *Amycolatopsis* genome as template. The *tktA* fragment was ligated into the *Bam*HI/*Nsi*I site of the plasmid pSET152-permE by an Assembly kit to construct the plasmid pSET152-tktA. The *ppsA* gene was amplified by PCR using VA-F14/VA-R14 as primers and *Amycolatopsis* genome as template. The *ppsA* fragment was ligated into the *Bam*HI*lNsi*I site of the plasmid pSET152-permE by an Assembly kit to construct the plasmid pSET152-ppsA.

pSET152-ppsA was digested with *Xba*I and *SpeI* and recovered the *ppsA* fragment from gel. The *ppsA* fragment was ligated to the *SpeI* site of pKG1132-*pyk-2500* by T4 ligase to construct the plasmid pKG1132*-pyk-ppsA.*

### 2. Construction of Amycolatopsis HM-154 integrating ppsA gene

The plasmids pSET152-tktA and pSET152-ppsA were respectively transformed into *Amycolatopsis* HM-151 by using the intergeneric conjugation method. The single colonies growing on the GYM solid medium with apramycin resistance were respectively strains HM-152 integrated with the *tktA* gene and HM-153 integrated with the *ppsA* gene.

The plasmid *pKG1132-pyk-ppsA* was transformed into *Amycolatopsis* HM-151 by using the intergeneric conjugation method. The single colony that grew on the GYM solid medium with apramycin resistance was the strain that had undergone homologous single exchange. The single-exchange strain was subcultured in the non-antibiotic GYM medium. The strain that grew on the non-antibiotic GYM plate but not on the apramycin resistance plate was the strain that had undergone homologous double exchange. The double-exchange strain was verified by PCR using the genome of *Amycolatopsis* HM-151 as a control. The primers for verifying the deletion *of pyk* gene were *pyk-*F1/*ppsA-*R1 and *ppsA-*F1/*pyk*-R2. The correct bands verified by PCR were sent for sequencing, and the correct sequencing strain was the *Amycolatopsis* HM-154 integrating *ppsA* gene. The PCR verification results of the HM-154 strain are shown in Figure 6.

### 3. Fermentation comparison of the strains HM-151, HM-152, HM-153 and HM-154

Fermentation experiments were conducted using *Amycolatopsis* HM-151, HM-152 and HM-153 strains, respectively, following the same fermentation experimental methods as in Example 1. The fermentation results are shown in Table 6.

**Table 6 Shake flask fermentation results of Amycolatopsis HM-151, HM-152 and HM-153**

| Strains | Vanillin concentration (g/L) |
|---|---|
| *Amycolatopsis* HM-151 | 3.85 |
| HM-152 | 3.65 |
| HM-153 | 4.69 |

The results indicated that the vanillin yield of strain HM-152, which has the integrated *tktA* gene, decreased by 5.2% compared to strain HM-151. In contrast, the vanillin yield of strain HM-153, which has the integrated *ppsA* gene, increased by 21.82% compared to strain HM-151.

Overexpression of *tktA* can enhance the flux of the precursor erythrose-4-phosphate (E4P), while overexpression of *ppsA* increases the flux of the precursor phosphoenolpyruvate (PEP). Both E4P and PEP are important precursors for vanillin synthesis, as shown in Figure 1.

The results showed that, although E4P and PEP are crucial precursors for vanillin synthesis, the expression of the transketolase gene *tktA* in *Amycolatopsis* did not enhance the vanillin yield when glucose is used as a substrate. In contrast, the phosphoenolpyruvate synthase gene *ppsA* can further increase the vanillin yield. This implies that *tktA* may not be a key gene for enhancing E4P flux in *Amycolatopsis.*

In summary, this invention involves knocking out the regulatory protein gene *tyrR1* and the pyruvate kinase gene *pyk* in engineered *Amycolatopsis* strains. Additionally, it overexpresses the endogenous chorismate mutase/prephenate dehydrogenase bifunctional enzyme gene *tyrA,* 3-deoxy-7-phosphoheptulonate synthase gene *aroF* and phosphoenolpyruvate synthase gene *ppsA,* resulting in the HM-154 strain. Through fermentation experiments, the vanillin yield of strain HM-154 reached 4.72 g/L, which is a 22.6% increase compared to strain HM-151 and a 277.6% increase compared to the starting strain HM-145. Furthermore, a small amount of by-products, including p-hydroxybenzaldehyde and protocatechuic acid, were detected in strain HM-153, with yields of 0.32 g/L and 0.15 g/L, respectively. Therefore, further research is needed to eliminate these by-products.

## Claims

1. An application of the deletion of regulatory protein gene *tyrR1* in *Amycolatopsis* sp. for improving the vanillin yield through fermentation with glucose as the substrate, with the gene sequence of *tyrR1* shown as SEQ ID NO:1 and its encoded protein accession number being WP_020416143.1.

2. An application of overexpressing *tyrA-aroF* genes and deleting regulatory protein *tyrR1* in *Amycolatopsis* for improving the vanillin yield through fermentation with glucose as the substrate, wherein *tyrA* encodes the chorismate mutase/prephenate dehydrogenase bifunctional enzyme with the nucleotide sequence shown as SEQ ID NO: 2 and the protein accession number WP_020419478.1, *aroF* encodes 3-deoxy-7-phosphoheptulonate synthase with the nucleotide sequence shown as SEQ ID NO:3 and the protein accession number WP_020418838.1, and *tyrR1* is represented by SEQ ID NO:1 with protein accession number WP_020416143.1.

3. A recombinant *Amycolatopsis* with high vanillin yield, which knocks out the regulatory protein gene *tyrR1* and the pyruvate kinase gene *pyk,* while overexpresses the endogenous chorismate mutase/prephenate dehydrogenase bifunctional enzyme gene *tyrA,* the 3-deoxy-7-phosphoheptulonate synthase gene *aroF,* and the phosphoenolpyruvate synthase gene *ppsA,* with the nucleotide sequences of genes *tyrR1, tyrA, aroF, pyk,* and *ppsA* shown as SEQ ID NO:1, SEQ ID NO:2, SEQ ID NO:3, SEQ ID NO:4, and SEQ ID NO:5, respectively.

4. A method of constructing the recombinant *Amycolatopsis* according to claim 3, wherein the method comprises the following steps:
(1) Construction of recombinant *Amycolatopsis* with *tyrR1* gene deletion
PCR amplifying upstream and downstream homologous arms of *tyrR1* gene by using VA-F2/VA-R2 and VA-F3/VA-R3 as primers and *Amycolatopsis* genome as template, and simultaneously connecting the two obtained *tyrRl-up-2500* and tyrRl-down-2500 fragments to the *Hind*III/*Eco*RI site of the plasmid pKG1132 by an Assembly kit to construct the plasmid pKG1132-*tyrR1*-2500;
transforming the obtained plasmid pKG1132-tyrRl-2500 into the starting strain of *Amycolatopsis* by using the intergeneric conjugation method, wherein the single colony that grows on the GYM solid medium with resistance to apramycin is the strain that has undergone homologous single exchange; carrying out subculture on the single-exchange strain in non-antibiotic GYM medium, wherein the strain that grows on non-antibiotic GYM plate but not on apramycin-resistant plate is the strain that has undergone homologous double exchange; conducting PCR verification on the double-exchange strain by using the genome of *Amycolatopsis* HM-145 as a control, wherein the correct strain is the recombinant *Amycolatopsis* with *tyrR1* gene deletion;
(2) Construction of recombinant *Amycolatopsis* integrating *tyrA* and *aroF* genes
PCR amplifying the *permE* fragment by using VA-F1/VA-R1 as primers and the plasmid pKC1139-permE as template, and connecting the obtained *permE* fragment to the *Pvu*I/*Eco*RV site of the plasmid pSET152 by an Assembly kit to construct the plasmid pSET152-permE; PCR amplifying the *tyrA* gene using VA-F8/VA-R8 as primers and *Amycolatopsis* genome as template, connecting the obtained *tyrA* fragment to the *Bam*HI/*Nsi*I site of the plasmid pSET152-permE by an Assembly kit to construct the plasmid pSET152-tyrA; PCR amplifying the *aroF* gene using VA-F10/VA-R10 as primers and *Amycolatopsis* genome as template, connecting the obtained *aroF* fragment to the *Bam*HI*lNsi*I site of the plasmid pSET152-permE by an Assembly kit to construct the plasmid pSET152-*aroF;*
digesting pSET152-aroF with *Xba*I and *SpeI* and recovering the *permE-aroF* fragment from gel, then connecting the *permE-aroF* fragment to the *SpeI* site of pSET152-tyrA by T4 ligase to construct a plasmid pSET152*-tyrA-aroF;* digesting *pSET 152-tyrA-aroF* with *Xba*I and *SpeI* and recovering the *tyrA-aroF* fragment from gel, then connecting *tyrA-aroF* fragment to the *SpeI* site of pKG1132-*tyrR1-*2500 by T4 ligase to construct the plasmid *pKG1132-tyrRl-tyrA-aroF;*
transforming the obtained plasmid *pKG1132-tyrRl-tyrA-aroF* into the recombinant *Amycolatopsis* with *tyrR1* gene deletion obtained in step (1) by using the intergeneric conjugation method, wherein the single colony that grows on the GYM solid medium with apramycin resistance is the strain that has undergone homologous single exchange; carrying out subculture on the single-exchange strain in the non-antibiotic GYM medium, wherein the strain that grows on the non-antibiotic GYM plate but not on apramycin-resistant plate is the strain that has undergone homologous double exchange; conducting PCR verifying on the double-exchange strain using the recombinant *Amycolatopsis* genome with *tyrR1* gene deletion in step (1) as a control, wherein the correct strain is the recombinant *Amycolatopsis* with *tyrR1* gene deletion and the *tyrA-aroF* gene integration;
(3) Construction of recombinant *Amycolatopsis* with *pyk* gene deletion
PCR amplifying upstream and downstream homologous arms of *pyk* gene using VA-F11/VA-R11 and VA-F12/VA-R12 as primers and *Amycolatopsis* genome as template, and simultaneously connecting the two obtained pyk-up-2500 and pyk-down-2500 fragments to the *HindIII*/*EcoRI* site of the plasmid pKG1132 by an Assembly kit to construct the plasmid pKG1132-pyk-2500;
transforming the plasmid pKG1132-pyk-2500 into the recombinant *Amycolatopsis* obtained in step (2) by using the intergeneric conjugation method, wherein the single colony that grows on the GYM solid medium with apramycin resistance is the strain that has undergone homologous single exchange; subculturing the single-exchange strain in the non-antibiotic GYM medium, wherein the strain that grows on the non-antibiotic GYM plate but not on the apramycin-resistant plate is the strain that has undergone homologous double exchange; conducting PCR verification on the double-exchange strain by using the recombinant *Amycolatopsis* genome obtained in step (2) as a control, wherein the correct strain is the recombinant *Amycolatopsis* with *tyrR1* and *pyk* gene deletion and the *tyrA-aroF* gene integration;
(4) Construction of recombinant *Amycolatopsis* with *ppsA* gene integrated at *pyk* site
PCR amplifying the *ppsA* gene by using VA-F14/VA-R14 as primers and *Amycolatopsis* genome as template, connecting the *ppsA* fragment to the *Bam*HI*lNsi*I site of the plasmid pSET152-permE by an Assembly kit to construct the plasmid pSET152-ppsA; then digesting pSET152-ppsA with *Xba*I and *Spe*I*,* recovering the *ppsA* fragment from gel, and connecting the *ppsA* fragment to the *SpeI* site of pKG1132-pyk-2500 by T4 ligase to construct the plasmid pKG1132-*pyk-ppsA;*
transforming the obtained plasmid pKG1132-pyk-ppsA into the recombinant *Amycolatopsis* HM-151 by using the intergeneric conjugation method, wherein the single colony that grows on the GYM solid medium with apramycin resistance is the strain that has undergone homologous single exchange; subculturing the single-exchange strain in the non-antibiotic GYM medium, wherein the strain that grows on the non-antibiotic GYM plate but not on the apramycin-resistant plate is the strain that has undergone homologous double exchange; conducting PCR verification on the double-exchange strain by using the *Amycolatopsis* HM-151 genome as a control, wherein the correct strain is the recombinant *Amycolatopsis* with high vanillin yield.

5. The application of the recombinant *Amycolatopsis* described in claim 3 or the recombinant *Amycolatopsis* obtained by the construction method described in claim 4 in the production of vanillin.

6. The application according to claim 5, **characterized by** inoculating the recombinant *Amycolatopsis* into 50 mL of seed culture medium M1, culturing at 30 °C and 200 rpm for 72 hours, then inoculating the seed culture into a 250 mL Erlenmeyer flask containing 50 mL of fermentation medium M1 at a 5% inoculation ratio, and fermenting at 37 °C and 200 rpm for 72 hours, wherein the M1 medium contains 25 g/L glucose, 10 g/L yeast extract, 0.8 g/L sodium chloride, 5 g/L potassium dihydrogen phosphate, 0.2 g/L magnesium sulfate heptahydrate, 0.05 g/L calcium chloride, with the rest being water, and the pH adjusted to 7.2.
